# EUROPEAN PATENT SPECIFICATION

(11) **EP 4 423 229 B1**
(45) Date of publication and mention of the grant of the patent: **01.04.2026**
(21) Application number: 22803021.9
(22) Date of filing: 20.10.2022
(51) Int. Cl.: C12G 3/025, A23C 11/10, A23C 9/127

(54) **SYMBIOTIC CULTURE OF BACTERIA AND YEAST FOR PRODUCTION OF WATER KEFIR**
SYMBIOTISCHE BAKTERIENKULTUR UND HEFE ZUR HERSTELLUNG VON WASSERKEFIR
CULTURE SYMBIOTIQUE DE BACTÉRIES ET DE LEVURE POUR LA PRODUCTION DE KÉFIR À L'EAU

(30) Priority: 29.10.2021 EP 21205736
(43) Date of publication of application: 04.09.2024
(73) Proprietor: Société des Produits Nestlé S.A., 1800 Vevey (CH)
(72) Inventor: DUBOUX, Stéphane, 1162 St-Prex (CH); ZHANG, Jiyuan, 1006 Lausanne (CH); ARAGÃO BÖRNER, Rosa, 1066 Epalinges (CH); PRIOUR, Sarah, 1010 Lausanne (CH); TAN, Jan Patrick, 1004 Lausanne (CH)
(74) Representative: Zingle, Catherine
(86) International application number: PCT/EP2022/079173
(87) International publication number: WO 2023/072715

(56) References cited:
- EP-A1- 2 878 683
- US-A1- 2015 224 158
- US-A1- 2019 343 152
- US-A1- 2020 352 190
- FABRICIO MARIANA FENSTERSEIFER ET AL: "Effect of freeze-dried kombucha culture on microbial composition and assessment of metabolic dynamics during fermentation", FOOD MICROBIOLOGY, ACADEMIC PRESS LTD, LONDON, GB, vol. 101, 23 August 2021 (2021-08-23), XP086798420, ISSN: 0740-0020, [retrieved on 20210823], DOI: 10.1016/J.FM.2021.103889
- GUZEL-SEYDIM ZEYNEP B ET AL: "A comparison of milk kefir and water kefir: Physical, chemical, microbiological and functional properties", TRENDS IN FOOD SCIENCE AND TECHNOLOGY, ELSEVIER SCIENCE PUBLISHERS, GB, vol. 113, 6 May 2021 (2021-05-06), pages 42 - 53, XP086602348, ISSN: 0924-2244, [retrieved on 20210506], DOI: 10.1016/J.TIFS.2021.04.041
- SMIT BART A ET AL: "Branched chain aldehydes: production and breakdown pathways and relevance for flavour in foods", APPLIED MICROBIOLOGY AND BIOTECHNOLOGY, SPRINGER BERLIN HEIDELBERG, BERLIN/HEIDELBERG, vol. 81, no. 6, 1 January 2009 (2009-01-01), pages 987 - 999, XP037215865, ISSN: 0175-7598, [retrieved on 20090101], DOI: 10.1007/S00253-008-1758-X

## Description

### Background of the invention

Water kefir is becoming very popular due to its perception as a natural and healthy vegan beverage. Industrial production of water kefir is very challenging, as it relies on the subculturing of an undefined mix of bacteria and yeast. The symbiotic nature of the microbial consortium, the relative heterogeneity in the community structure over the world, and the lack of flavor knowledge have all made water kefir starter culture development complicated.

In US 2015/224158 A1 (HOULE C [CA] ET AL), plant extracts which have been fermented with kefir grains are described, as well as methods of production of these extracts, and the use of these fermented extracts in animal and human health.

In US 2019/343152 A1 (KUHL D [CA] ET AL), a method for making kombucha alcohol is described. The method includes a preparing a symbiotic culture, fermenting a sweetened tea with the symbiotic culture to provide a fermented tea drink, and combining an alcohol beverage to the fermented tea drink. The method for making kombucha alcohol is reported to be useful for combining the properties associated with alcohol and Kombucha for human consumption.

Fabricio et al., in Food Microbiology, 101, 2022, 103889 (Epub 2021 Aug 23), describe the correlation between the microbial community and metabolites evolution with fermentation in a traditionally produced kombucha by evaluating the microbial community and the main metabolites produced. The effects of starter cultures processed in three different ways (control, starter culture without liquid suspension (CSC), and a freeze-dried starter culture (FDSC)) on changes in kombucha composition, such as antioxidant activity and sensory analysis are described. It is reported that there were no statistically significant differences in the acceptance test in sensory analysis.

In EP 2878683 A1 (UNIV LATVIJAS [LV]), the production of fermented polyfunctional synbiotic beverages for mass consumption is described, specifically a microbial culture association that consists of individual cultures of Lactobacillus plantarum LUMBI B78 and Saccharomyces cerevisiae LUMBI R42 in a ratio of 2:1 is described for the production of fermented beverages.

In US 2020/352190 A1 (JOHNSON M [US] ET AL), a non-dairy fermented water kefir base composition is described, as well as methods of making and/or using the non-dairy fermented water kefir base composition to produce nutritional products, such as food and beverage products.

Guzel-Seydim et al., in Trends in Food Science & Technology, 2021, 113(4), compare milk kefir (MK) grains and water kefir (WK) grains and examine microbiological, chemical, and functional properties. The fermented beverages produced from these grains are reported to have different physical and chemical characteristics and different microbiological composition.

Smit et al., in Appl Microbiol Biotechnol. 2009, 81(6):987-99, review aspects influencing the formation of branched aldehydes, such as 2-methyl propanal and 2- and 3-methyl butanal. These are reported to be important flavour compounds in many food products, both fermented and non-fermented (heat-treated) products.

There are no good starter cultures existing for water kefir. Some companies are using mixes of strains to produce a fermented product, but these are relatively far away from a traditional water kefir in terms of organoleptic properties.

### Embodiments of the invention

The invention relates in general to a fermented beverage composition, comprising gluconic acid and lactic acid, wherein the concentration ratio of gluconic acid to lactic acid is greater than 1, preferably between 2 to 10, or between 3 to 9, or between 4 to 8, or between 5 to 7, or about 6, wherein the fermented beverage composition further comprises *Zymomonas mobilis,* lactic acid bacteria and *Saccharomyces,* wherein the *Zymomonas mobilis* is the most abundant microorganism.

In some embodiments, the beverage composition further comprises ethanol, wherein the ethanol is present at a concentration less than 10 g/L, or less than 5 g/L, or less than 4 g/L, or less than 3 g/L, or less than 2 g/L, or less than 1 g/L. In some embodiments, the ethanol is present at a concentration of between 1 to 10 g/L, or between 1 to 5 g/L, or between 1 to 4 g/L, or between 1 to 3 g/L, or between 1 to 2 g/L.

In some embodiments, the beverage composition further comprises sugar, wherein the sugar is present at a concentration less than 50 g/L, preferably less than 40 g/L, preferably less than 30 g/L, preferably less than 20 g/L, preferably less than 10 g/L. In some embodiments, the sugar is present at a concentration of between 1 to 50 g/L, or between 1 to 40 g/L, or between 1 to 30 g/L, or between 1 to 20 g/L, or between 1 to 10 g/L. Preferably, the sugar is cane sugar.

In one embodiment, the beverage composition further comprises 2-phenylethanol, 3-methlylbutanol, and 3-methylbutanal at a concentration of greater than 3 mg/L. Preferably, greater than 50% of the 2-phenylethanol and 3-methlylbutanol is produced by yeast, preferably greater than 60%, preferably greater than 70%. Preferably greater than 50% of the methylbutanal is produced by Zymomonas, preferably greater than 60%, preferably greater than 70%.

In one embodiment, 2-phenylethanol, 3-methlylbutanol, and 3-methylbutanal are each present at a concentration of at least 1 mg/L in the beverage composition.

In one embodiment, the beverage composition further comprises *Bifidobacterium.*

In one embodiment, the lactic acid bacteria is selected from the group consisting of *Liquorilactobacillus, Lactiplantibacillus* and combinations of these.

In one embodiment, the beverage composition comprises
a. *Zymomonas mobilis;* and
b. *Liquorilactobacillus hordei;* and
c. *Saccharomyces cerevisiae* and/or *Saccharomyces bayanus;* and
d. *Bifidobacterium aquikefiri.*

In one embodiment, the beverage composition comprises *Zymomonas mobilis;* and Liquorilactobacillus *hordei;* and Saccharomyces *cerevisiae;* and *Bifidobacterium aquikefiri.*

In one embodiment, the beverage composition comprises *Zymomonas mobilis;* and *Liquorilactobacillus hordei;* and *Saccharomyces bayanus;* and *Bifidobacterium aquikefiri.*

In one embodiment, the beverage composition comprises fewer than 10 species of bacteria and yeast, or fewer than 9, or fewer than 8, or fewer than 7, or fewer than 6, or fewer than 5 species of bacteria and yeast. In one embodiment, the beverage composition comprises fewer than 3 species of yeast.

The invention further relates to a starter culture for preparing a fermented beverage composition, wherein said starter culture comprises
a. *Zymomonas mobilis;* and
b. Lactic acid bacteria, for example *Liquorilactobacillus hordei;* and
c. *Saccharomyces,* for example *Saccharomyces bayanus,*
wherein the *Zymomonas mobilis* is the most abundant microorganism In one embodiment, the starter culture has an inoculation ratio of colony forming units of *Zymomonas mobilis* to colony forming units of *Saccharomyces* of between 1 to 18, for example between 2 to 10, for example about 6.

In one embodiment, the starter culture has an inoculation ratio of colony forming units of lactic acid bacteria to colony forming units of *Saccharomyces* of between 8 to 22, For example between 10 to 20, for example between 12 to 18.

In one embodiment, the starter culture has an inoculation ratio of colony forming units of *Zymomonas mobilis* to colony forming units of lactic acid bacteria of between 0.1 to 3, for example between 0.5 to 2, for example between 0.8 to 1.5, for example about 1.2.

In one embodiment, the starter culture further comprises *Bifidobacterium,* for example *Bifidobacterium aquikefiri.*

In one embodiment, the *Bifidobacterium aquikefiri* is CNCM I-5756.

In one embodiment, the *Zymomonas mobilis* is CNCM I-5755.

In one embodiment, the *Liquorilactobacillus hordei* is CNCM I-5757.

In one embodiment, the *Saccharomyces bayanus* is CNCM I-5758.

In one embodiment, the starter culture comprises fewer than 10 species of bacteria and yeast, or fewer than 9, or fewer than 8, or fewer than 7, or fewer than 6, or fewer than 5 species of bacteria and yeast. In one embodiment, the beverage composition comprises fewer than 3 species of yeast.

The invention further relates to a method of preparing a beverage composition, said method comprising fermenting an aqueous solution comprising sucrose with a starter culture according to the invention.

In one embodiment, the aqueous solution comprises fruit or vegetable extracts, for example fruit or vegetable extracts, for example fruit or vegetable juice or pulp.

In one embodiment, the method comprises a microbial inactivation step and/or a drying step.

### Detailed description of the invention

### Fermentation

In one embodiment, the fermentation was performed for about 30 hours. This solution may comprise about 5% sucrose. Fermentation may be carried out in two phases. In the first phase, the incubation may be carried out for about 24 hours at about 30 degrees centigrade. The conditions may be completely aerobic. In a second phase, the incubation may be carried out for about 6 hours.

### Amplicon sequencing and shotgun metagenome sequencing

Extracted DNA may be processed by, for example, amplicon sequencing or shotgun whole genome sequencing.

Amplicon sequencing may involve the use of universal primers targeting the full length 16S and ITS1/ITS2 regions for bacteria and fungi. The resulting amplicon pool may be sequenced using a PacBio platform, after which the resulting quality checked reads may be denoised by DADA2. Taxonomical classification may be performed by SPINGO, for example against the databases SILVA 138 and UNITE 8.2 for 16S and ITS reads respectively. Resulting read counts may then be normalized for 16S copy number variation by manually crosschecking against the database rrnDB.

Shotgun metagenome sequencing may be performed in an Illumina platform. Resulting reads may be taxonomically classified using the metagenome classifier Kaiju using the NCNI nonredundant database.

Isolation of microbiota may be done by plating diluted liquor or homogenized grain suspension in media, for example MRS, HHD, YPD, PCA, or DMA. Incubation may be performed, for example between two to five days.

Variants of bacteria and fungi may be identified by SPINGO classification.

### Flavor profiles

Sugars, organic acids, amino acids, and volatile compounds can be measured as described herein. For example, carbohydrates may be quantified using a DIONEX HPAEC-PAD system with sucrose, glucose, and fructose as standards. Organic acids may be quantified by LC-MSMS. Volatile analysis may be performed through HS-SPME/GC-MS untargeted and targeted analysis. Targeted analysis may be done on the above fermentation markers through external calibration.

### Co-culture construction

Strains may be revived from glycerol stocks. After between 16 to 40 hours of growth, resulting cultures may be subcultured in an appropriate medium volume and incubated for between 16 to 24 hours. Prior to fermenting, the cell density may be measured from the OD600. The co-culture strain concentrations used, in CFU/ml, may be about the same as those shown in example 2. The appropriate volume of each pre culture for each ratio can be combined. Cells may be pelleted, washed and resuspended in sucrose solution followed by inoculating in a medium of sucrose solution and fig extract. Fermentations may take place statically in aerobic conditions, for example at about 35 hours at about 30°C.

Carbohydrates, organic acids, and alcohols may be quantified by HPLC coupled with a refractive index and UV detector, for example as described herein. Organic acids may be quantified via HPLC coupled with triple quadruple mass spectrometry, for example as described herein.

Targeted profiling of flavor/aroma associated volatiles may be conducted by headspace solid phase microextraction coupled with gas chromatogram and mass spectrometry, for example as described herein. Compounds may be identified based on retention time and MS spectrum, and quantification may be performed through external calibration with a pure standard mix.

The ethanol content may be between 0.1 to 0.7%, for example between 0.38 to 0.63% alcohol by volume.

The beverage composition of the invention is preferably vegan. The beverage composition preferably does not comprise dairy products, for example milk. The beverage composition preferably comprises greater than 40% *Zymomonas mobilis* as a percentage of total microbial cells in the beverage composition, preferably greater than 50%, preferably greater than 60%. The beverage composition preferably comprises less than 95% *Zymomonas mobilis* as a percentage of total microbial cells, preferably Less than 90%, preferably less than 85%, preferably less than 80%. Preferably the beverage composition comprises about 75% *Zymomonas mobilis* as a percentage of total microbial cells in the beverage composition.

In one embodiment, the volatiles present in the greatest amount are fusel alcohols. The fusel alcohols may comprise 2-methylbutanol, 2-phenylethanoland fusel aldehydes, for example 2-methylbutanal, 3-methylbutanal, 2-phenylacetaldeyde.

In one embodiment, ethyl acetate is the ester present in the greatest amount amongst the esters present.

In one embodiment, the most abundant organic acid is gluconic acid. In one embodiment, the second most abundant organic acid is lactic acid.

*Zymomonas mobilis* is the most abundant microorganism.

In one embodiment, *Saccharomyces bayanus* is the least abundant microorganism.

Final *Z. mobilis* levels may be between 7.6 to 7.9 logs. Final *L. hordei* levels may be between 7.1-7.6 logs. Final *B. aquikefiri* may be between 7.4 to 7.8 logs. Final *S*. *bayanus* levels may be between 6.4 to 6.8 logs.

In one embodiment, *Saccharomyces* is a non-trivial yeast of the co-culture.

The following microorganisms were deposited with the Collection Nationale de Cultures de Microorganismes (CNCM), Institut Pasteur, 25 rue du Docteur Roux, F-75724 PARIS Cedex 15, France, and have a CNCM deposit number and date of deposit as shown, where applicable. *Bifidobacterium aquikefiri* (CNCM I-5756, date of deposit 15.10.2021)*. Zymomonas mobilis* (CNCM I-5755, date of deposit 15.10.2021). *Liquorilactobacillus hordei* (CNCM I-5757, date of deposit 19.10.2021). *Saccharomyces bayanus* (CNCM I-5758, date of deposit 19.10.2021).

As used herein, the term "about" is understood to refer to numbers in a range of numerals, for example the range of -30% to +30% of the referenced number, or -20% to +20% of the referenced number, or -10% to +10% of the referenced number, or -5% to +5% of the referenced number, or -1% to +1% of the referenced number. All numerical ranges herein should be understood to include all integers, whole or fractions, within the range.

As used herein, the term "vegan" refers to an edible composition which is entirely devoid of animal products, or animal derived products, for example eggs, milk, honey, fish, and meat.

As used herein, the term "vegetarian" relates to an edible composition which is entirely devoid of meat, poultry, game, fish, shellfish or by-products of animal slaughter.

Those skilled in the art will understand that they can freely combine all features of the present invention disclosed herein. In particular, features described for the compositions of the present invention may be combined with the method or uses of the present invention and vice versa. Further, features described for different embodiments of the present invention may be combined. Where known equivalents exist to specific features, such equivalents are incorporated as if specifically referred to in this specification.

Further advantages and features of the present invention are apparent from the figures and non-limiting examples.

### Examples

### Example 1

### Water kefir community profiling

Fermentation. The fermentation of water kefir (WK) was followed for 30 hours in biological triplicates. For each setup, 10.7%(w/w) of WK grains were inoculated in 80.5%(w/w) sucrose (5%) solution and 8.8%(w/w) backslop. Fermentation was carried out in two phases. In the first phase, grains were incubated for 24 hours at 30 °C carried out in aerobic conditions. The grains were then sieved out and the jars tightly capped for the second phase, after which the fermentation was left to continue for an additional 6 hours. Samples were collected after the specified time points from the beginning (T0) until the end of fermentation (T30) and either immediately analyzed or stored at -20 °C until needed.

DNA extraction. WK grains were washed with 1X phosphate-buffered saline (PBS) before being resuspended in 1X PBS and homogenized to obtain a liquid suspension. The WK liquor was taken as is. DNA extraction was performed using the ZymoBIOMICS DNA/RNA kit (Zymo Research) with the grain suspension and liquor as inputs according to manufacturer's instructions. Extracted DNA was processed in 2 ways: amplicon sequencing and shotgun whole genome sequencing.

Amplicon sequencing. Universal primers targeting the full length 16S and ITS1/ITS2 regions for bacteria and fungi were used. The resulting amplicon pool was sequenced using a PacBio platform, after which the resulting quality-checked reads were denoised by DADA2 and taxonomically classified by SPINGO against the databases SILVA 138 and UNITE 8.2 for 16S and ITS reads, respectively. Resulting read counts were then normalized for 16S copy number variation by manually cross-checking against the database rrnDB.

Shotgun metagenome sequencing. An 800 bp library was constructed and paired-end sequencing was performed in an Illumina platform in 2x250 bp mode. Resulting reads were similarly quality-checked and taxonomic classification performed using the metagenome classifier Kaiju using the NCBI non-redundant database.

Isolation. WK microbiota were isolated by plating diluted WK liquor and homogenized grain suspension in various rich and selective media: MRS, HHD, YPD, PCA, DMS. Incubation was performed for 2-5 days. Isolated colonies were identified either through MALDI-TOF (Bruker) of fresh colonies or through Sanger sequencing of amplified 16S and ITS1/ITS2 regions for bacteria and fungi, respectively.

### Results

The microbial community profile of water kefir grain and liquor was analysed. Amplicon sequencing of 16S (Table 1) and ITS1-ITS4 (Table 2) at the start of fermentation; Table 3 shows shotgun sequencing of metagenomes at the start (0h) and end (30h) of fermentation. Error bars represent the standard deviation of 3 biological replicates.

**Table 1. Amplicon sequencing of 16S at the start of fermentation. Values are expressed as relative abundance of each taxa.**

| Taxa | Liquor | Grain |
|---|---|---|
| *Zymomonas mobilis* | 6.50E-01 | 6.81E-03 |
| *Bifidobacterium aquikefiri* | 2.81E-01 | 7.19E-03 |
| *Liquorilactobacillus nagelii* | 2.77E-02 | 4.07E-03 |
| *Liquorilactobacillus satsumensis* | 1.84E-02 | 3.83E-03 |
| *Liquorilactobacillus mali* | 2.06E-02 | 2.28E-03 |
| *Lentilactobacillus hilgardii* | 0.00E+00 | 3.28E-03 |
| *Lacticaseibacillus paracasei* | 5.56E-04 | 2.74E-04 |
| *Schleiferilactobacillus perolens* | 4.46E-04 | 6.94E-04 |
| *Acetobacter persici* | 6.38E-04 | 0.00E+00 |
| *Acetobacter orientalis* | 1.95E-04 | 0.00E+00 |

**Table 2. Amplicon sequencing of ITS1-ITS4 at the start of fermentation. Values are expressed as relative abundance of each taxa.**

| Taxa | Liquor | Grain |
|---|---|---|
| *Saccharomyces cerevisiae* | 6.14E-01 | 2.43E-02 |
| *Saccharomyces bayanus* | 4.21E-02 | 6.46E-04 |
| *Dekkera anomala* | 3.34E-01 | 2.39E-02 |
| *Dekkera bruxellensis* | 1.65E-05 | 6.57E-05 |
| *Pichia membranifaciens* | 1.00E-02 | 1.13E-04 |
| *Pichia fermentans* | 1.42E-04 | 3.69E-05 |

**Table 3. Shotgun sequencing of metagenomes at the start (0h) and end (30h) of fermentation. Values are expressed as relative abundance of each taxa.**

| Taxa | Grain (0h) | Grain (24h) | Liquor (0h) | Liquor (30h) |
|---|---|---|---|---|
| *Zymomonas mobilis* | 8.14E-01 | 7.92E-01 | 7.53E-01 | 7.17E-01 |
| *Bifidobacterium aquikefiri* | 6.22E-02 | 8.13E-02 | 5.20E-02 | 9.41E-02 |
| *Liquorilactobacillus satsumensis* | 2.52E-02 | 2.62E-02 | 1.08E-02 | 3.83E-03 |
| *Liquorilactobacillus hordei* | 2.31E-02 | 2.44E-02 | 2.67E-03 | 1.52E-03 |
| *Dekkera bruxellensis* | 1.53E-02 | 1.81E-02 | 4.66E-02 | 7.18E-02 |
| *Liquorilactobacillus nagelii* | 1.29E-02 | 1.04E-02 | 8.06E-03 | 4.95E-03 |
| *Leuconostoc suionicum* | 9.30E-03 | 9.59E-03 | 8.72E-03 | 5.81E-03 |
| *Oenococcus kitaharae* | 8.97E-03 | 9.15E-03 | 1.38E-02 | 8.36E-03 |
| *Acetobacter indonesiensis* | 4.82E-03 | 5.12E-03 | 3.76E-03 | 4.46E-03 |
| *Lentilactobacillus hilgardii* | 4.29E-03 | 4.87E-03 | 1.19E-02 | 9.82E-03 |
| *Saccharomyces cerevisiae* | 4.26E-03 | 3.81E-03 | 9.39E-03 | 4.33E-03 |
| *Lactiplantibacillus plantarum* | 2.99E-03 | 3.37E-03 | 5.34E-02 | 5.82E-02 |
| *Lacticaseibacillus paracasei* | 2.84E-03 | 2.78E-03 | 1.62E-04 | 8.50E-05 |
| *Saccharomyces eubayanus* | 2.73E-03 | 2.11E-03 | 5.29E-03 | 2.36E-03 |
| *Liquorilactobacillus mali* | 2.06E-03 | 2.07E-03 | 3.73E-03 | 4.18E-03 |
| *Komagataeibacter hansenii* | 2.04E-03 | 2.05E-03 | 1.95E-03 | 1.73E-03 |
| *Leuconostoc mesenteroides* | 1.79E-03 | 1.46E-03 | 1.14E-03 | 5.72E-04 |
| *Torulaspora delbrueckii* | 7.96E-04 | 6.12E-04 | 5.22E-03 | 5.88E-03 |
| *Leuconostoc pseudomesenteroides* | 6.62E-04 | 5.01E-04 | 8.00E-04 | 7.23E-04 |
| *Curvibacter sp. PAE-UM* | 9.36E-05 | 1.16E-04 | 7.68E-03 | 3.81E-04 |

SPINGO classification identified 11 and 6 sequence variants for bacteria and fungi, respectively, at T0 (Table 1)*. Zymomonas mobilis* comprised majority of the bacterial community with 65.0% of assigned reads. This is followed by *Bifidobacterium aquikefiri (28.1%), Lactobacillus nagelii (2.7%), Lactobacillus mali* (2.0%), and *Lactobacillus satsumensis* (1.8%). Other *Lactobacillus* and *Acetobacter* were present at percentages less than one percent. *Saccharomyces cerevisiae* (61.4%) comprised majority of the fungal community, followed by *Dekkera anomala* (33.4%), *Saccharomyces bayanus* (4.2%), and *Pichia membranifaciens* (1.0%.) (Table 2).

Taxonomic classification by Kaiju identified a total of 20 species (13 genus) with relative abundance levels >0.1% (Table 3). Relative species proportions are similar to those obtained from amplicon sequencing. The higher sensitivity to low-abundance species can be attributed to a less biased shotgun approach that minimizes the likely amplification skewing in an already naturally skewed community. In the liquor, the community is dominated by *Z. mobilis,* comprising 75.3% of assigned reads, followed by *Lactobacillus hordei* (5.3%), *Bifidobacterium aquikefiri* (5.2%), and *Dekkera bruxellensis* (4.7%). Lactic acid bacteria (11.5%) have the highest richness in the community with 11 species in the 6 genera *Leuconostoc, Oenococcus, Lacticaseibacillus, Lactiplantibacillus, Lentilactobacillus,* and *Liquorilactobacillus.* Acetic acid bacteria are present in relatively lower proportions of 0.6% and are comprised of species from the *Acetobacteraceae* family: *Acetobacter indonesiensis* and *Komagataeibacter hansenii.* Reads assigned to fungal species (0.66%) all fall under the family *Saccharomycetaceae* and are split between *Dekkera bruxellensis, Saccharomyces cerevisiae, Saccharomyces eubayanus,* and *Torulaspora delbrueckii.*

While the grain community appears to be stable over time, some shifts in overall percentages are observed in the liquor (Table 3). Almost all species decreased in abundance, with the largest seen in *Z. mobilis* with an apparent decrease from 75.3 to 71.7%. Increases were mostly seen on microorganisms that were already relatively abundant at the beginning of fermentation, with *L. hordei* (5.3→5.8%), *B. aquikefiri* (5.2→9.4%), and *D. bruxellensis* (4.7→7.1%) showing the largest increases. Overall assigned reads to LAB decreased slightly from 11.5 to 10% while AAB levels remained relatively stable.

**Table 3a. shows the intersection between species isolated and identified from culture-independent profiling and those isolated.**

| Both (isolates + DNA sequencing) | Isolates only | | DNA sequencing only | |
|---|---|---|---|---|
| *Bacteria* | | | | |
| *Acetobacter orientalis* | *L. paracasei* | *Acetobacter tropicalis* | *Acetobacter indonesiensis* | *L. plantarum* |
| *Acetobacter persici* | *L. satsumensis* | | *Curvibacter sp.* | *Le. suinocum* |
| *Bifidobacterium aquikefiri* | *Lc. mesenteroides*/ *pseudomensenteroides* | | *Komagataeibacter hansenii* | *Oenococcus kitaharae* |
| *L. hordei*/*mali* | *Zymomonas mobilis* | | *L. hilgardii* | |
| *L. nagelii* | | | *L. perolens* | |

| Yeast | | | | |
|---|---|---|---|---|
| *S.cerevisiae* | *Dekkera bruxellensis* | | *Dekkera anomala* | *Torulaspora delbrueckii* |
| *S. bayanus* | | | *S. eubayanus* | *Pichia fermentans* |

Isolation on various selective media recovered a total of 13-15 species which overall comprise 97-99% of the kefir liquor community at T0. Consolidating both amplicon and shotgun sequencing results show 11 species which were not able to be isolated. This incongruence is likely due to the natively low amounts (all less than 1%) these species were present in WK liquor or due to fastidious nutritional requirements which are absent in the media used.

### Example 2

### Flavor profiles of water kefir

For the measurement of sugars, organic acids, amino acids, and volatile compounds, the water kefir liquor was centrifuged (13,000 xg, 15 minutes 4 °C) and the supernatant filtered through a 0.2 µm filter. Samples for organic acids and carbohydrates were diluted 1:10 and 1:1000 before analysis; samples for volatile analysis were diluted 1:1 with HCl to normalize all samples to the lowest pH. Volatile analysis was performed through HS-SPME/GC-MS untargeted and targeted analysis. Targeted analysis was done on 20 fermentation markers through external calibration. Organic acids were quantified through LC-MSMS with 7 standards (malic, citric, quinic, gluconic, lactic, succinic, fumaric). Carbohydrates were quantified using a Dionex HPAEC-PAD system with sucrose, glucose, and fructose as standards. Quantification of ethanol and acetic acid were performed with HPLC-RI/UV.

**Table 4. Temporal metabolite dynamics of water kefir.**

| Metabolites | | Fermentation time point (h) | | | | |
|---|---|---|---|---|---|---|
| | | 0 | 8 | 16 | 24 | 30 |
| Carbohydrates (g/L) | | | | | | |
| | Sucrose | 36.870 | 24.893 | 21.793 | 9.917 | 11.820 |
| | Glucose | 1.987 | 4.080 | 2.853 | 1.843 | 1.577 |
| | Fructose | 2.653 | 8.420 | 10.233 | 10.783 | 10.363 |

| Organic acids (g/L) | | | | | | |
|---|---|---|---|---|---|---|
| | Citric | 0.010 | 0.010 | 0.010 | 0.010 | 0.010 |
| | Fumaric | 0.025 | 0.025 | 0.025 | 0.025 | 0.025 |
| | Gluconic | 0.371 | 2.615 | 2.407 | 2.650 | 2.059 |
| | Lactic | 0.118 | 0.173 | 0.215 | 0.254 | 0.267 |
| | Malic | 0.004 | 0.005 | 0.005 | 0.005 | 0.005 |
| | Succinic | 0.042 | 0.055 | 0.059 | 0.070 | 0.073 |
| | Acetic | 0.324 | 0.599 | 0.753 | 0.897 | 1.072 |
| | Ethanol (g/L) | 3.342 | 4.898 | 7.419 | 9.886 | 11.770 |

| Flavor-associated volatiles (mg/L) | | | | | | |
|---|---|---|---|---|---|---|
| | 1-Octanol | 0.013 | 0.013 | 0.013 | 0.013 | 0.013 |
| | 2-Methylbutanal | 0.033 | 0.180 | 0.032 | 0.025 | 0.029 |
| | 2-Methylbutanol | 1.031 | 1.291 | 1.368 | 1.563 | 1.527 |
| | 2-Phenylacetaldehyde | 0.021 | 0.028 | 0.021 | 0.019 | 0.019 |
| | 2-Phenylethanol | 0.289 | 0.395 | 0.788 | 0.545 | 0.540 |
| | 2-Phenylethyl acetate | 0.010 | 0.010 | 0.010 | 0.010 | 0.010 |
| | 3-Methylbutanal | 0.013 | 0.032 | 0.006 | 0.001 | 0.000 |
| | 3-Methylbutanol | 1.061 | 1.281 | 1.362 | 1.712 | 1.524 |
| | Benzaldehyde | 0.005 | 0.005 | 0.004 | 0.004 | 0.004 |
| | Benzyl acetate | 0.008 | 0.008 | 0.008 | 0.008 | 0.008 |
| | Ethyl acetate | 0.256 | 0.510 | 0.676 | 0.471 | 0.596 |
| | Furfuryl acetate | 0.006 | 0.005 | 0.005 | 0.006 | 0.006 |
| | Isobutyl acetate | 0.003 | 0.003 | 0.002 | 0.002 | 0.002 |
| | Isopentyl acetate | 0.001 | 0.001 | 0.001 | 0.001 | 0.001 |

Total carbohydrates dropped 42.7% (4.16→2.38%) after fermentation. Sucrose levels dropped 68% (3.69→1.18%) for 24 hours before stabilizing. Glucose was the lowest carbohydrate throughout fermentation, peaking at 0.41% after 8 hours before steadily dropping to 0.16% at the end. Fructose levels increased from 0.26% to a peak of 1.0% in 24 hours before stabilizing.

Gluconic acid appears to be the most abundant acid in WK. Concentrations increased 4.6-fold from an initial concentration of 0.37 g/L to a final concentration of 2.06 g/L. Acetic acid, the second most abundant acid in WK, increased from 0.32 to 1.07 g/L and is noted to be above the odor threshold. Lactic and succinic acid steadily increased throughout fermentation. Lactic acid levels finished at 0.27 g/L from an initial of 0.12 g/L-a 56% increase. Succinic acid is present in relatively lower proportions; a 75% increase from 0.04 to 0.07 g/L. All other acids were present below 0.02 g/L and remained static over 30 hours.

The fusel alcohols (2-methylbutanol, 3-methylbutanol, 2-phenylethanol) and fusel aldehydes (2-methylbutanal, 3-methylbutanal, 2-phenylacetaldeyde) appear to be key volatiles instead of esters. Fusel alcohols are present at higher intensity and above the odor threshold as indicated in literature. Fusel aldehydes are present at lower intensity but are above the odor threshold. These volatiles are known to be produced by yeasts (and certain bacteria), which were confirmed through single-strain screening. Ethyl acetate is the main ester present in water kefir among the ester group. Ethanol levels increase from 3.23 g/L to 10.71 g/L for a final ABV of 1.36%.

### Example 3

### Co-culture construction

### Methods

Each strain was revived from glycerol stocks in 5-10 mL of culture. After 16-40 h of growth, resulting cultures were used to generate the pre-culture by subculturing at 0.5-1% (v/v) in the appropriate medium volume and incubating for 16-24 hours. Prior to fermenting, the cell density was computed from the OD600 using a previously calculated CFU/mL-OD600 linear regression.

**Table 5: Strain concentrations per co-culture in CFU/mL**

| Co-culture | Strain | | | | |
|---|---|---|---|---|---|
| | *Zymomonas mobilis* 1 (ZM1) | *Liquorilactobacill us hordei* 5 (LH5) | *Saccharomyces bayanus* 1 (SB1) | *Bifidobacterium aquikefiri* 1 (BA1) | *Dekkera bruxellensis 6* (DB6) |
| **MR** | 1.70E+07 | 4.00E+06 | 8.00E+05 | 6.00E+05 | 0 |
| **ER** | 2.20E+06 | 4.40E+06 | 3.67E+05 | 1.10E+06 | 0 |
| **no ZM** | 0 | 4.40E+06 | 3.67E+05 | 1.10E+06 | 0 |
| **no LH** | 2.20E+06 | 0 | 3.67E+05 | 1.10E+06 | 0 |
| **no SB** | 2.20E+06 | 4.40E+06 | 0 | 1.10E+06 | 0 |
| **no BA** | 2.20E+06 | 4.40E+06 | 3.67E+05 | 0 | 0 |
| **Ratio 3** | 1.10E+06 | 4.40E+06 | 3.67E+05 | 0 | 0 |
| **Ratio 10** | 3.67E+06 | 4.40E+06 | 3.67E+05 | 0 | 0 |
| **DBmix** | 2.20E+06 | 4.40E+06 | 0 | 0 | 3.67E+05 |

The appropriate volume of each pre-culture for each ratio was combined (Table 5). Cells were pelleted and washed with PBS, before resuspending in sucrose solution and inoculating in a medium of 99% (w/w) sucrose solution and 1% fig extract. Fermentations took place statically in loosely capped bottles for 35 hours at 30°C.

### Microbial enumeration of co-cultures

To track the growth of the individual strains, the concentration of each strain in each co-culture at the beginning and end of fermentation was measured. Cell suspensions were aliquoted and serially diluted in PBS. Spotting was then done by plating 10 µL of the dilutions in 3-5 replicates on the appropriate agar medium and incubated as listed on Table 6.

**Table 6. Incubation conditions for selective growth of co-culture strains.**

| Strain | Agar medium | Antibiotic/antimycotic | Oxygen conditions | Incubation time^{b} (d) |
|---|---|---|---|---|
| LH5 | MRS | 21.6 µg/mL natamycin | AN | 2 |
| SB1 | YPD | 100 µg/mL chlortetracycline | AE | 1.5 |
| ZM1 | DSMZ 1445¹ | 21.6 µg/mL natamycin | AN | 2 |
| BA1 | MRS + 0.05% cysteine-HCl | 21.6 µg/mL natamycin | AN | 3 |
| | | 50 µg/mL mupirocin | | |
| AP1 | mDMS² no deoxycholate, pH 6.0 | 21.6 µg/mL natamycin | AE | 2 |
| DB6 | YPD | 100 µg/mL chlortetracycline | AE | 3 |
| | | 10 µg/mL cycloheximide | | |

| | | | | |
|---|---|---|---|---|
| a - AE -aerobic; AN - anaerobic b - All strains are incubated at 30°C | | | | |

### Sensory evaluation

A blinded sensory evaluation was performed by trained panel by rating the samples relative to a reference sample. Scoring of the attributes-acid, bitter, sweet, fruity, floral, fermented, vinegar, fizzy-was done on rate-all-that-applies format, with each applicable attribute scored on an intensity scale from 1 (slightly) to 5 (extremely).

### Metabolite profiling

For liquid chromatography, samples were centrifuged (21,000 x g, 15 min, 4°C), passed through a 0.2 µm filter, and diluted with Milli-Q water (Millipore, Buchs, Switzerland) as needed. To monitor the concentrations of key fermentation compounds, carbohydrates (sucrose, glucose, fructose), organic acids (gluconic, lactic, citric, acetic, succinic, propionic, butyric), and alcohols (ethanol, glycerol) were quantified by HPLC coupled with a refractive index and UV detector (HPLC-RI/UV, Agilent Technologies AG, Basel, Switzerland) using an Aminex HPX-78H column (Bio-Rad Laboratories AG, Cressier, Switzerland). Compounds from 20 µL of injected volume were separated isocratically with 5 mM H2SO4 for 45 min with a flow rate of 0.6 mL/min and column temperature of 35°C. Peaks were integrated and quantified using external standards via ChemStation (Agilent Technologies).

Organic acids (citric, fumaric, gluconic, lactic, malic, quinic, succinic) were quantified via HPLC coupled with triple quadrupole mass spectrometry (Triple Quad 6460, Agilent) using an Eclipse XDB-C18 column (5 µm, 4.6x150 mm, Agilent). The mobile phase was composed of Solvent A (0.1% (v/v) formic acid in milli-Q water) and solvent B (0.1% (v/v) formic acid in acetonitrile). Flow rate was 0.7 mL/min and injection volume 1 µL. Electrospray ionization was operated in negative ionization mode. MassHunter (Agilent Technologies) was used to integrate and obtain the relative peak area.

Targeted profiling of flavor/aroma-associated volatiles (FAV) was conducted by headspace solid-phase microextraction coupled with gas chromatogram and mass spectrometry One milliliter of sample was directly pipetted inside a 10 mL glass headspace vial. All vials were maintained at 6°C before extraction. The sample was first incubated for 10 min at 30°C, and extracted with a polydimethylsiloxane/divinylbenzene fiber for 10 min at 30°C, after which it was injected in to GC for separation on a DB-WAX column (Agilent Technologies; 30 m, 0.25 mm, 0.25 µm). Compounds were identified based on retention time and MS spectrum, and quantification was performed through external calibration with a pure standard mix.

### Sensorial similarity between water kefir and starter culture

The sensory attributes of co-cultures relative to a natural WK reference is shown. Accounting for their relative abundance in the natural WK community, their ability to replicate the complex interactions necessary to sustain community growth, and their capacity to produce the relevant WK metabolites, four strains were selected for the design of co-cultures: *Zymomonas mobilis 1 (ZM1), Saccharomyces bayanus 1 (SB1), Liquorilactobacillus hordei 5 (LH5),* and *Bifidobacterium aquikefiri 1 (BA1).* Two ratios were constructed: (1) Metagenomics-based ratio (MR)- in which the cell concentrations were approximated from metagenomic sequencing results; and (2) Experiment-based ratio (ER) - which was developed through iterative testing.

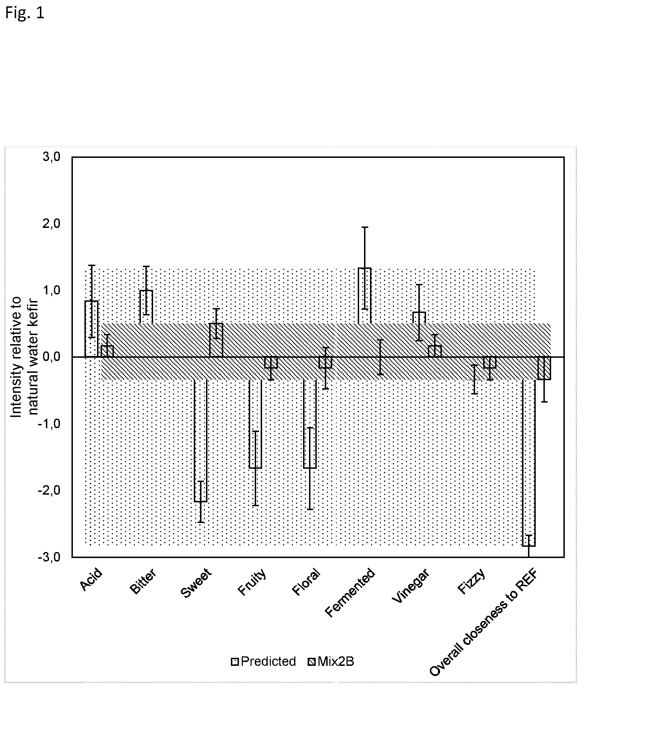

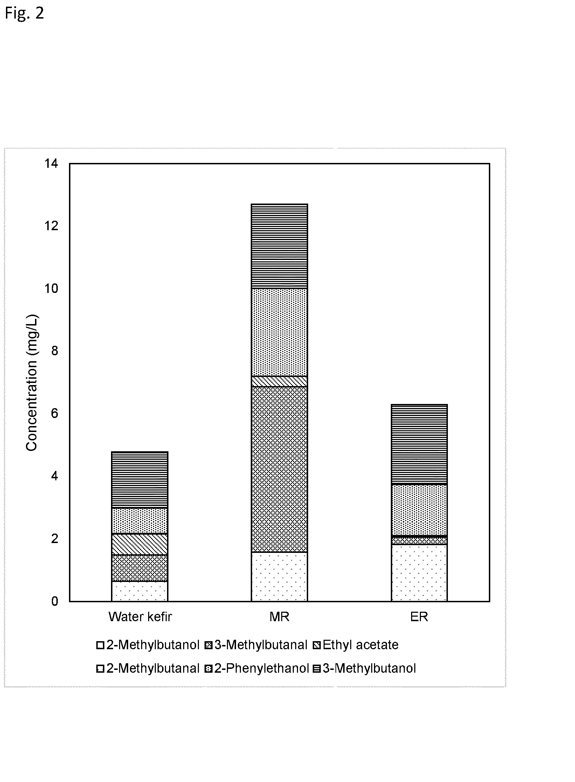

ER scored similarly to the natural WK reference on the sensory attributes tested, while the predicted ratio was marked with more fermented and less fruity notes and overall deviated from the global natural WK sensory profile (Figure 1). Volatile profiling corroborates this result, with an overall higher similarity between the natural water kefir and ER (Figure 2).

Figure 2 shows quantification of flavor-associated volatile compounds of co-cultures and natural water kefir. Only compounds with a concentration greater than 0.1 mg/L were shown. MR - Metagenomics-based ratio; ER - Experiment-based ratio.

The experimentally derived water kefir co-culture is chemically characterized by low sugar, low ethanol, fusel alcohols and high gluconic acid.

Figure 3 shows quantification of carbohydrates and ethanol after fermentation.

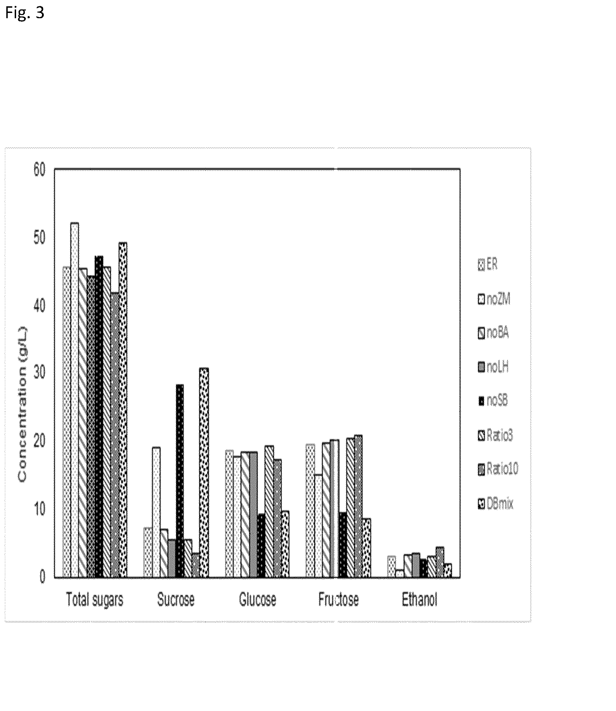

The relevant compounds of ER were quantified to assess the chemical profile of the product. Total sugar content is measured at 45 g/L, with the sucrose depleted from an initial 50 g/L to 7.4 g/L (Figure 3). Ethanol content is at 3 g/L and can go up to 5 g/L, corresponding to an alcohol by volume (ABV) of 0.38-0.63% (Figure 3).

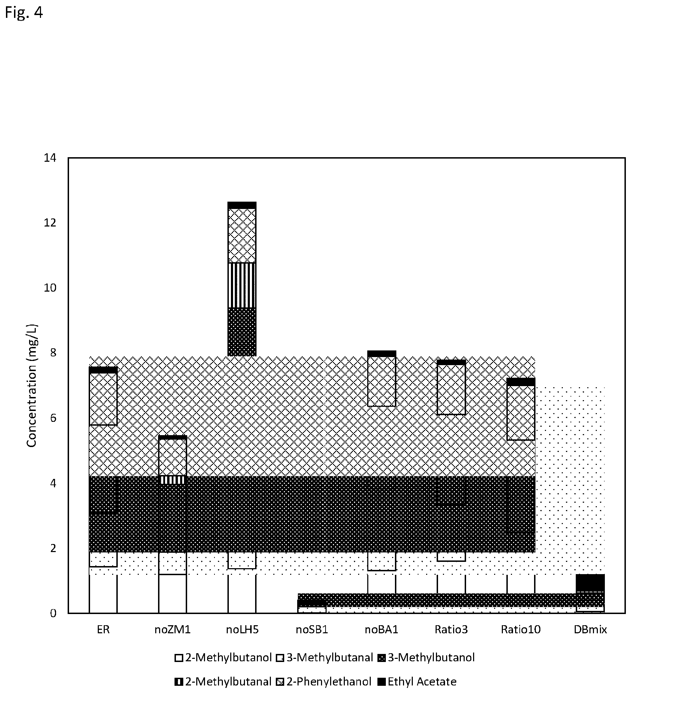

Figure 4 shows quantification of flavor-associated volatile compounds after fermentation. Only compounds with a concentration greater than 0.1 mg/L were shown.

Through targeted volatile profiling using typical fermented beverage markers, fusel alcohols (2-methylbutanol, 2-phenylethanol) and fusel aldehydes (2-methylbutanal, 3-methylbutanal, 2-phenylacetaldeyde) appear to be the key volatiles for the water kefir beverage composition of the invention, instead of esters (Figure 4). Fusel alcohols are present at higher intensity and above the odor threshold as indicated in literature. Fusel aldehydes are present at lower intensity but are above the odor threshold. These volatiles are known to be produced by yeasts (and certain bacteria), which were confirmed through single-strain screening (not shown). Ethyl acetate is the main ester present in water kefir among the ester group.

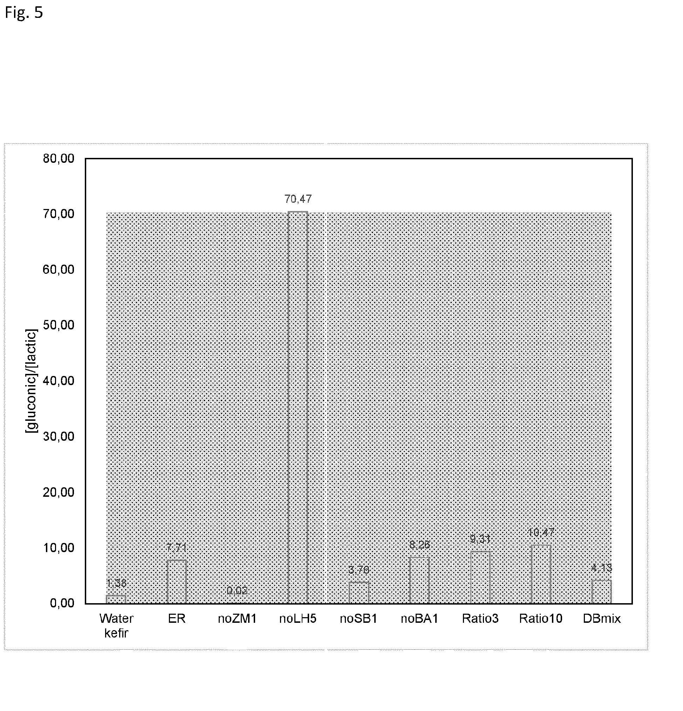

Figure 5 shows the ratio of gluconic acid and lactic acid concentration in mg/mL after fermentation. Like the natural water kefir (Figure 5), the most abundant organic acid ER is gluconic acid and followed by lactic acid. The absolute concentration of each organic slightly differs from batch-to-batch, yet the ratio between the two stays around the range of 5-8 (Figure 5).

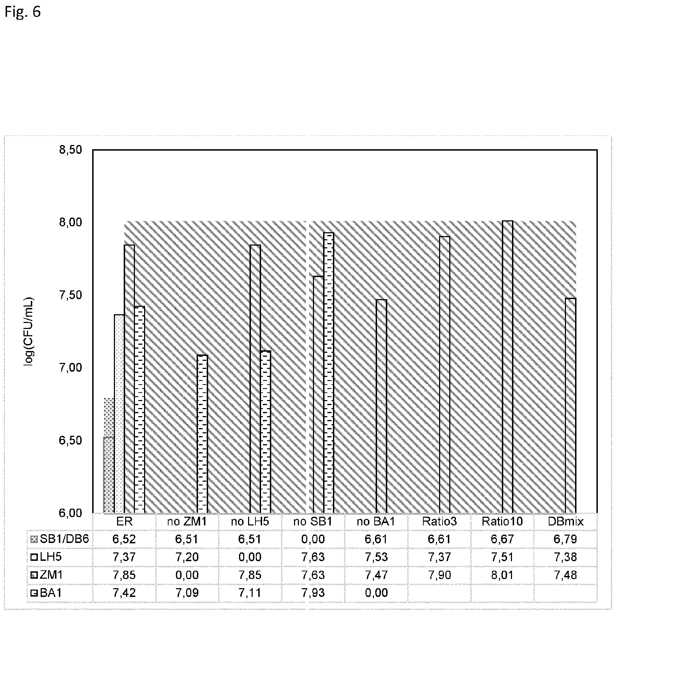

Figure 6 shows final colony forming concentrations of each strain after fermentation. Values are expressed in log10(CFU/mL). All strains of the co-culture experience a 1 log increase after a 35-hour fermentation, with ZM1 and SB1 being the most and least abundant microorganism at the end (Figure 6). Final ZM1 levels are between 7.6 to 7.9 logs; final LH5 levels between 7.1-7.6; BA1 at 7.4-7.8; and SB1 at 6.4-6.8. Removal of individual starter culture strains compromises water kefir organoleptic and microbial properties.

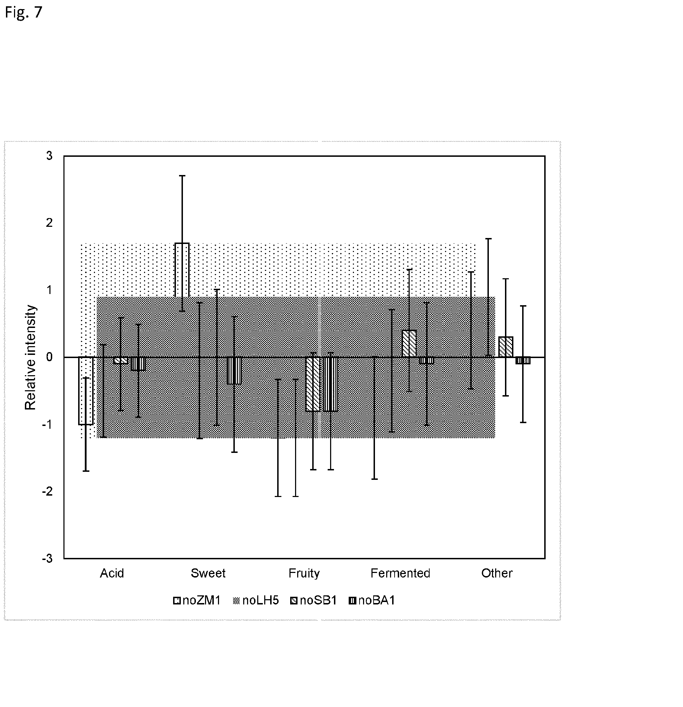

Figure 7 shows relative sensory intensity of minus-one co-cultures relative to ER. Error bars represent the Least significant difference at 5%.

To assess the individual impact of each strain in the ER co-culture, minus-one fermentations were performed (Table 5). Sensory results indicate the removal of ZM1 to significantly reduce the acidity and fermented notes and increase sweetness as a result (Figure 7). On the other hand, both ZM1 and LH5 appear to be the main contributors of fruitiness notes in the co-culture. Removing ZM1, LH5, and SB1 strongly alters the resulting acid and volatile profile, with minimal effect upon removing BA1 (Figure 4, Figure 5). These chemical differences are explained by the different final strain densities after fermentation. Specifically, removing ZM1 reduces the levels of BA1 and LH5, while removing BA1 reduces ZM1 levels relative to ER (Figure 6).

The starting ZM1 amount is essential in maintaining the sensory attributes of the starter culture.

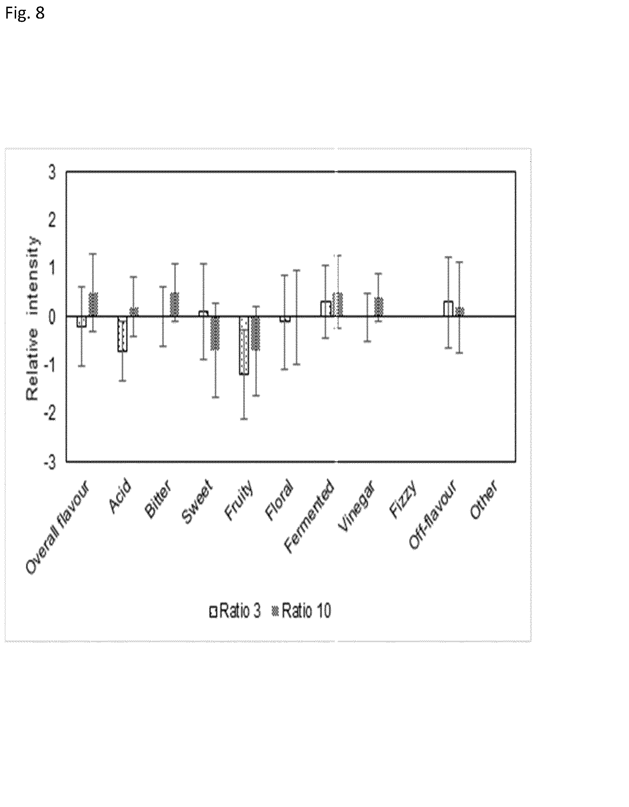

Figure 8 shows relative sensory intensity of co-cultures with varying inoculating ratios of ZM1:SB1 relative to ER. Error bars represent the Least significant difference at 5%.

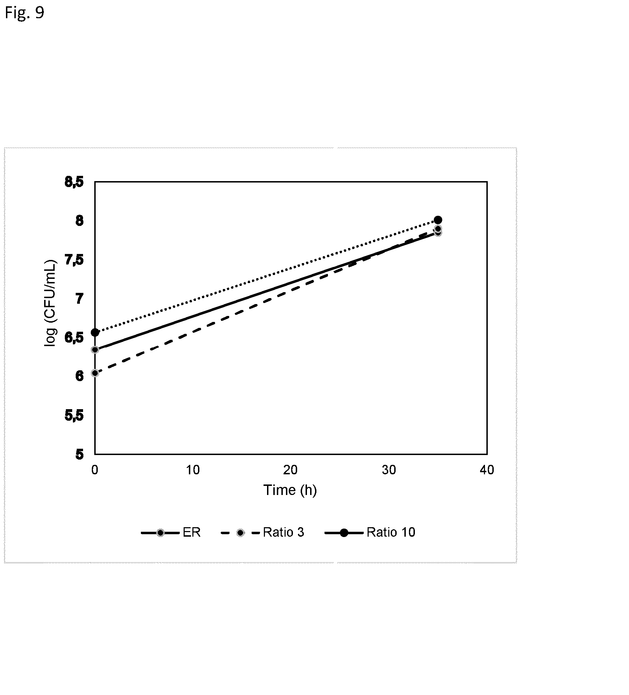

To assess the impact of changing the initial strain amounts in the final organoleptic properties of the product, two co-cultures with an initial ZM1:SB1 ratio of 3 and 10 were designed (Table 5). Ratio 3 had significantly decreased acidity and fruitiness relative to the ER, while Ratio 10 had a noted increase in bitter and vinegar attributes (Figure 8). The decrease in fruitiness notes in Ratio 3 is attributed to the lowered growth of LH5 in Ratio 3 versus Ratio 10 (Figure 6). Ratio 10 exhibited a divergent volatile profile and had an overall higher level of gluconic and lactic acid relative to ER (Figure 4, Figure 5). Interestingly, ZM1 levels at the end of fermentation were similar between Ratio 3, Ratio 10, and ER despite a 0.3 log10 difference in initial inoculating amounts (Figure 9). Overall, these results highlight the importance of the initial levels of ZM1 in influencing the organoleptic properties of the product.

Figure 9 shows initial and final cell density of ZM1 at varying inoculation densities.

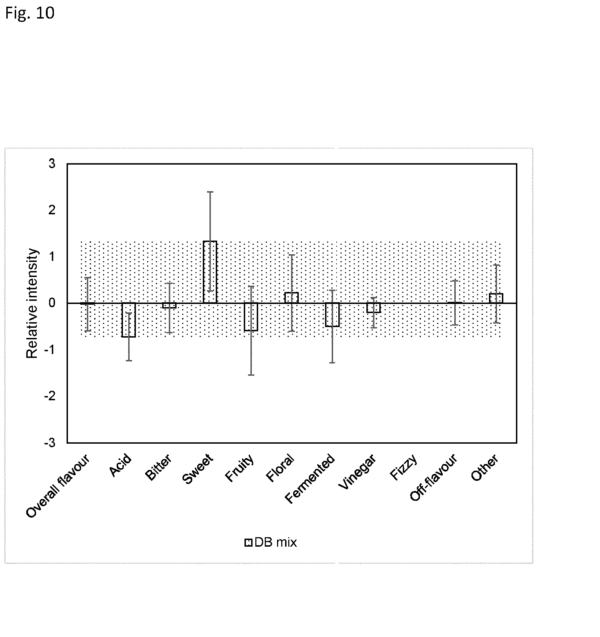

Saccharomyces is a non-trivial yeast of the co-culture. To investigate whether SB1 could be replaced by other yeasts known to be present in natural water kefir, SB1 was exchanged by Dekkera bruxellensis 6 (DB6)-also isolated from the same water kefir. The resulting product-called DBmix-was sweeter and notably less acidic than Mix 2B (Figure 10). In addition, the volatile profile of the product was vastly different, with lower levels of fusel alcohols and aldehydes (Figure 4). Lastly, the replacement of SB1 impaired the growth of ZM1 (Figure 6), resulting in lower final gluconic acid levels (Figure 4), which shows relative sensory intensity of DBmix relative to ER. Error bars represent the Least significant difference at 5%.

## Claims

1. A fermented beverage composition comprising gluconic acid and lactic acid, wherein the concentration ratio of gluconic acid to lactic acid is greater than 1, and wherein the fermented beverage composition further comprises *Zymomonas mobilis,* lactic acid bacteria and *Saccharomyces,* wherein the *Zymomonas mobilis* is the most abundant microorganism.

2. The beverage composition according to claim 1, wherein the concentration ratio of gluconic acid to lactic acid is between 2 to 10.

3. The beverage composition according to claim 1 or claim 2, wherein the beverage composition further comprises ethanol, wherein the ethanol is present at a concentration less than 10 g/L, for example less than 5 g/L.

4. The beverage composition according to claim 1 to 3, wherein the beverage composition further comprises sugar, wherein the sugar is present at a concentration less than 50 g/L.

5. The beverage composition according to claim 1 to 4, wherein the beverage composition further comprises 2-phenylethanol, 3-methlylbutanol, and 3-methylbutanal at a combined concentration of greater than 3 mg/L.

6. The beverage composition according to claim 1 to 5, further comprising *Bifidobacterium.*

7. The beverage composition according to any one of claims 1 to 6 wherein the beverage composition comprises fewer than 10 species of bacteria and yeast.

8. A starter culture for preparing a fermented beverage composition, wherein said starter culture comprises
a*. Zymomonas mobilis;* and
b. lactic acid bacteria, for example *Liquorilactobacillus hordei;* and
c*. Saccharomyces,* for example *Saccharomyces bayanus,* wherein the *Zymomonas mobilis* is the most abundant microorganism.

9. The starter culture according to claim 8, wherein the starter culture has an inoculation ratio of colony forming units of *Zymomonas mobilis* to colony forming units of *Saccharomyces* of between 1 to 18, for example between 2 to 10, for example about 6.

10. The starter culture according to claims 8 or 9, wherein the starter culture has an inoculation ratio of colony forming units of lactic acid bacteria to colony forming units of *Saccharomyces* of between 8 to 22.

11. The starter culture according to claims 8 to 10, wherein the starter culture has an inoculation ratio of colony forming units of *Zymomonas mobilis* to colony forming units of lactic acid bacteria of between 0.1 to 3, for example about 1.2.

12. The starter culture according to claims 8 to 11, wherein the starter culture comprises fewer than 10 species of bacteria and yeast.

13. A method of preparing a beverage composition, said method comprising fermenting an aqueous solution comprising sucrose with a starter culture according to any one of claims 8 to 12.

14. The method according to claim 13 wherein the aqueous solution comprises fruit or vegetable extracts, for example fruit or vegetable juice or pulp.

15. The method according to claim 13 or 14, said method comprising a microbial inactivation step and/or a drying step.

## Patentansprüche

1. Fermentierte Getränkzusammensetzung, umfassend Gluconsäure und Milchsäure, wobei das Konzentrationsverhältnis von Gluconsäure zu Milchsäure größer als 1 ist, und wobei die fermentierte Getränkzusammensetzung ferner *Zymomonas mobilis,* Milchsäurebakterien und *Saccharomyces* umfasst, wobei *Zymomonas mobilis* der am häufigsten vorkommende Mikroorganismus ist.

2. Getränkzusammensetzung nach Anspruch 1, wobei das Konzentrationsverhältnis von Gluconsäure zu Milchsäure zwischen 2 bis 10 liegt.

3. Getränkzusammensetzung nach Anspruch 1 oder 2, wobei die Getränkzusammensetzung ferner Ethanol umfasst, wobei das Ethanol in einer Konzentration von weniger als 10 g/l, zum Beispiel weniger als 5 g/l, vorliegt.

4. Getränkzusammensetzung nach Anspruch 1 bis 3, wobei die Getränkzusammensetzung ferner Zucker umfasst, wobei der Zucker in einer Konzentration von weniger als 50 g/l vorliegt.

5. Getränkzusammensetzung nach Anspruch 1 bis 4, wobei die Getränkzusammensetzung ferner 2-Phenylethanol, 3-Methylbutanol und 3-Methylbutanal in einer kombinierten Konzentration von mehr als 3 mg/l umfasst.

6. Getränkzusammensetzung nach Anspruch 1 bis 5, ferner umfassend *Bifidobacterium.*

7. Getränkzusammensetzung nach einem der Ansprüche 1 bis 6, wobei die Getränkzusammensetzung weniger als 10 Arten von Bakterien und Hefe umfasst.

8. Starterkultur zum Herstellen einer fermentierten Getränkzusammensetzung, wobei die Starterkultur umfasst
a. *Zymomonas mobilis; und*
b. Milchsäurebakterien, zum Beispiel *Liquorilactobacillus hordei;* und
c. *Saccharomyces,* zum Beispiel *Saccharomyces bayanus,* wobei *Zymomonas mobilis* der am häufigsten vorkommende Mikroorganismus ist.

9. Starterkultur nach Anspruch 8, wobei die Starterkultur ein Inokulationsverhältnis von koloniebildenden Einheiten *von Zymomonas mobilis zu* koloniebildenden Einheiten *von Saccharomyces* zwischen 1 zu 18 aufweist, zum Beispiel zwischen 2 zu 10, zum Beispiel etwa 6.

10. Starterkultur nach Anspruch 8 oder 9, wobei die Starterkultur ein Inokulationsverhältnis von koloniebildenden Einheiten von Milchsäurebakterien zu koloniebildenden Einheiten von *Saccharomyces* zwischen 8 bis 22 aufweist.

11. Starterkultur nach den Ansprüchen 8 bis 10, wobei die Starterkultur ein Inokulationsverhältnis von koloniebildenden Einheiten von *Zymomonas mobilis* zu koloniebildenden Einheiten von Milchsäurebakterien zwischen 0,1 bis 3 aufweist, zum Beispiel etwa 1,2.

12. Starterkultur nach den Ansprüchen 8 bis 11, wobei die Starterkultur weniger als 10 Arten von Bakterien und Hefe umfasst.

13. Verfahren zum Herstellen einer Getränkzusammensetzung, wobei das Verfahren das Fermentieren einer wässrigen Lösung, umfassend Saccharose mit einer Starterkultur gemäß einem der Ansprüche 8 bis 12.

14. Verfahren nach Anspruch 13, wobei die wässrige Lösung Frucht- oder Gemüseextrakte umfasst zum Beispiel Frucht- oder Gemüsesaft oder - fruchtfleisch, umfasst.

15. Verfahren nach Anspruch 13 oder 14, das Verfahren umfassend einen Schritt der mikrobiellen Inaktivierung und/oder einen Trocknungsschritt.

## Revendications

1. Composition de boisson fermentée comprenant de l'acide gluconique et de l'acide lactique, dans laquelle le rapport de concentration entre l'acide gluconique et l'acide lactique est supérieur à 1, et dans laquelle la composition de boisson fermentée comprend en outre *Zymomonas mobilis,* des bactéries d'acide lactique et *Saccharomyces,* dans laquelle *Zymomonas mobilis* est le micro-organisme le plus abondant.

2. Composition de boisson selon la revendication 1, dans laquelle le rapport de concentration entre l'acide gluconique et l'acide lactique est compris entre 2 et 10.

3. Composition de boisson selon la revendication 1 ou la revendication 2, dans laquelle la composition de boisson comprend en outre de l'éthanol, dans laquelle l'éthanol est présent à une concentration inférieure à 10 g/L, par exemple inférieure à 5 g/L.

4. Composition de boisson selon la revendication 1 à 3, dans laquelle la composition de boisson comprend en outre du sucre, dans laquelle le sucre est présent à une concentration inférieure à 50 g/L.

5. Composition de boisson selon la revendication 1 à 4, dans laquelle la composition de boisson comprend en outre du 2-phényléthanol, du 3-méthlylbutanol et du 3-méthylbutanal à une concentration combinée supérieure à 3 mg/L.

6. Composition de boisson selon la revendication 1 à 5, comprenant en outre *Bifidobacterium.*

7. Composition de boisson selon l'une quelconque des revendications 1 à 6, dans laquelle la composition de boisson comprend moins de 10 espèces de bactéries et de levures.

8. Culture de départ permettant de préparer une composition de boisson fermentée, dans laquelle ladite culture de départ comprend
a. *Zymomonas mobilis ;* et
b. des bactéries d'acide lactique, par exemple *Liquorilactobacillus hordei ;* et
c. *Saccharomyces,* par exemple *Saccharomyces bayanus,* dans laquelle *Zymomonas mobilis* est le micro-organisme le plus abondant.

9. Culture de départ selon la revendication 8, dans laquelle la culture de départ a un rapport d'inoculation entre des unités formant des colonies de *Zymomonas mobilis* et des unités formant des colonies de *Saccharomyces* compris entre 1 et 18, par exemple entre 2 et 10, par exemple environ 6.

10. Culture de départ selon les revendications 8 ou 9, dans laquelle la culture de départ a un rapport d'inoculation entre des unités formant des colonies de bactéries d'acide lactique et des unités formant des colonies de *Saccharomyces* compris entre 8 et 22.

11. Culture de départ selon les revendications 8 à 10, dans laquelle la culture de départ a un rapport d'inoculation entre des unités formant des colonies de *Zymomonas mobilis* et des unités formant des colonies de bactéries d'acide lactique compris entre 0,1 et 3, par exemple environ 1,2.

12. Culture de départ selon les revendications 8 à 11, dans laquelle la culture de départ comprend moins de 10 espèces de bactéries et de levures.

13. Procédé de préparation d'une composition de boisson, ledit procédé comprenant la fermentation d'une solution aqueuse comprenant du saccharose avec une culture de départ selon l'une quelconque des revendications 8 à 12.

14. Procédé selon la revendication 13, dans lequel la solution aqueuse comprend des extraits de fruits ou de légumes, par exemple du jus ou de la pulpe de fruits ou de légumes.

15. Procédé selon la revendication 13 ou 14, ledit procédé comprenant une étape d'inactivation microbienne et/ou une étape de séchage.
